# EUROPÄISCHE PATENTANMELDUNG

(11) **EP 0 856 505 A1**
(43) Veröffentlichungstag der Anmeldung: **05.08.1998**
(21) Anmeldenummer: 98101437.6
(22) Anmeldetag: 28.01.1998
(51) Int. Cl.: C07C 37/20, C07C 39/16

(54) **Verfahren zur selektiven Herstellung von2,2'Bis (4-Hydroxyphenyl) propanen**

(30) Priorität: 04.02.1997 DE 19703934
(71) Anmelder: Degussa Aktiengesellschaft, 60387 Frankfurt am Main (DE)
(72) Erfinder: Wieland, Stefan, Dr., 63069 Offenbach (DE); Gradl, Robert, 63755 Alzenau (DE); Röder, Stefan, 36391 Sinntal (DE)

(57) **Zusammenfassung**

Die Erfindung betrifft ein Verfahren zur selektiven Herstellung von 2,2'-Bis(4-hydroxyphenyl)propanen hoher Reinheit und einem hohen Isomerenverhältnis von p/p zu p/o-Produkt durch die Umsetzung von entsprechenden Phenolen mit Ketonen in der Gegenwart sulfonat- und mercaptogruppenhaltiger Organopolysiloxane nach dem Zusatz von Wasser.

## Beschreibung

Die Erfindung betrifft ein Verfahren zur selektiven Herstellung von 2,2'-Bis(4-hydroxyphenyl)propanen hoher Reinheit und einem hohen Isomerenverhältnis von p/p zu P/o-Produkt durch die Umsetzung von entsprechenden Phenolen mit Ketonen in der Gegenwart sulfonat- und mercaptogruppenhaltiger Organopolysiloxane. Beispielhaft sei die Reaktionsgleichung für die Synthese von Bisphenol-A (BPA) angegeben:

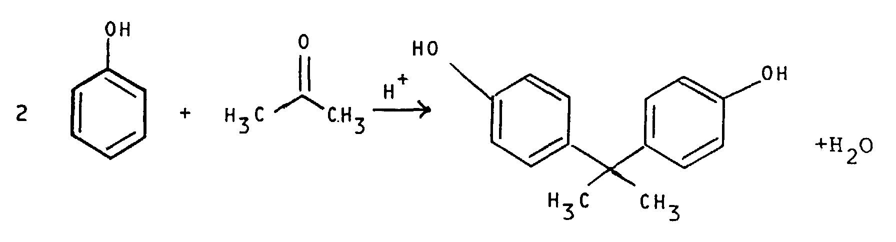

Der homogen mit Salzsäure katalysierte Prozeß ist heute weitgehend durch ein Verfahren mit organischen Ionenaustauschern auf Styrol/Divinylbenzolbasis abgelöst worden. Technisch arbeitet man bei dem heterogen katalysierten Verfahren ohne Lösungsmittel in einer 40 - 120°C heißen Schmelze, wobei Phenol und Aceton meist in einem Verhältnis von 10 : 1 bis 15 : 1 eingesetzt werden. da für die Weiterverarbeitung in Kunststoffen sehr hochreines Bisphenol-A zur Verfügung stehen muß und etwa die Hälfte der bei der Herstellung anfallenden Kosten durch die Reinigung des Produktes verursacht werden, strebt man einen möglichst selektiven Reaktionsverlauf bei hohen Umsätzen an Aceton an. Um Aktivität und Selektivität der heterogenen Katalysatoren zu steigern, verwendet man als Cokatalysatoren bifunktionelle Aminomercaptoverbindungen (z.B. Thiazoline, Mercaptoalkylamine), die über die Amingruppe an den Sulfonsäurerest des organischen Ionenaustauschers gebunden werden (DE-OS 37 27 641, US-PS 3,634,341). Vor Beginn der Reaktion müssen zunächst die sauren Ionenaustauscher in Phenol vorgequollen und anschließend mit den schwefelhaltigen Verbindungen modifiziert werden. Alle möglichen Belegungsgrade der Ionenaustauscher mit Mercaptogruppen sind patentiert worden, doch werden bevorzugt 5 - 30 mol% der Austauschkapazität des Harzes durch Mercaptoverbindungen modifiziert (EP-A1 0 620 041).

Die Herstellung des Bisphenol-A erfolgt in den meisten Fällen über ein kontinuierliches Verfahren, bei dem nach der Reaktion das Bisphenol-A mit Phenol als 1 : 1 Adukt auskristallisiert, von Phenol in verschiedenen Waschprozessen mit Wasser und organischen Lösungsmitteln befreit und als Reinkomponente erneut kristallisiert wird. Die Ausbeuten an Bisphenol-A betragen bei Verweilzeiten am Katalysator von bis zu 6 h zwischen 90 und 95 % bezogen auf die Unterschußkomponente Aceton bei Selektivitäten von 95 %. Das Hauptnebenprodukt ist 2,4'-Dihydroxyphenylpropan (o,p-Bisphenol-A). Weitere, nur in sehr geringer Menge anfallende Verbindungen, wie das 4-Hydroxyphenyl-2,2,4-trimethylchroman oder Triphenole, sind für die gelbe Farbe der Schmelze verantwortlich und ebenfalls unerwünscht.

Allerdings nimmt die Katalysatoraktivität und -selektivität der modifizierten organischen Ionenaustauscher bereits nach relativ kurzer Zeit ab. Eine Ursache ist die Abspaltung der Mercaptoverbindungen von der Oberfläche der sulfonsauren Ionenaustauscher (Desaktivierung der cokatalytischen Einheit).

Gemäß EP-A2 0 583 712 nimmt die Aktivität derartiger Katalysatoren in geringerem Umfang ab, wenn man dem Eduktgemisch aus Aceton und Phenolen 0,6 bis 5 Gew.-% Wasser hinzufügt. Gleichzeitig tritt ein geringfügiger Anstieg der Selektivität um maximal 0,5 % ein.

Im allgemeinen ist nach dem Stand der Technik jedoch davon auszugehen, daß bei Verwendung von organischen Ionenaustauschern als Katalysatoren die Selektivität in Gegenwart von Wasser sinkt (s. a. Vergleichsbeispiele).

Aus der deutschen Anmeldung 195 36 363.9 ist ein Verfahren zur Herstellung von substituierten Bisphenolen bekannt, bei dem man unter Einsatz sulfonat- und mercaptogruppenhaltiger Organopolysiloxane arbeitet. Diese Katalysatoren zeichnen sich dadurch aus, daß im Laufe der Umsetzung Aktivität und Selektivität nicht abnehmen, da keine Mercaptogruppen abgespalten werden. Geeignete Katalysatoren eines verwandten Typs werden in der DE 195 40 497.1 beschrieben.

Aufgabe der Erfindung ist es, ein Verfahren bereitzustellen, mit dessen Hilfe man die gewünschten Verbindungen mit erhöhter Selektivität gewinnt.

Die Offenbarung der beiden genannten deutschen Patentanmeldungen ist im Hinblick auf die dort beschriebenen Katalysatoren Inhalt der vorliegenden Erfindung.

Gegenstand der Erfindung ist ein Verfahren zur Herstellung von 2,2'-Bis(4-hydroxyphenyl)propanen der allgemeinen Formel 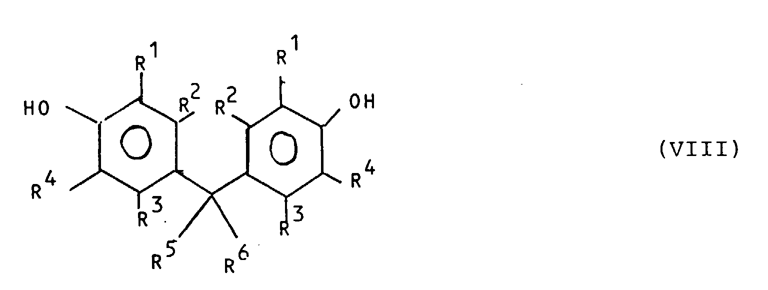 aus Phenolen der allgemeinen Formel 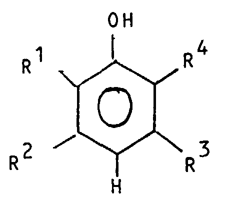 in der R¹, R², R³ und R⁴ unabhängig voneinander für Wasserstoff, C₁- bis C₄-Alkylgruppen, aromatische Substituenten oder Halogene wie F, Cl, Br und I stehen, bevorzugt, 2-Methylphenol (o-Kresol), 2,6-Dimethylphenol, 2-Ethylphenol, 1,3,5-Trimethylphenol(1,3,5-Xylenol), 2,3,5,6-Tetramethylphenol, 2-Phenylphenol, 2-Chlorphenol, 3-Chlorphenol, 2-Bromphenol, 2,6-Dichlorphenol, insbesondere Phenol, durch Umsetzung mit Carbonylverbindungen der allgemeinen Formel 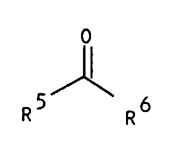 in der R⁵ und R⁶ unabhängig voneinander Wasserstoff, C₁ bis C₆-Alkyl, C₆ bis C₁₀-Cycloalkyl, C₃ bis C₁₄-Aryl, C₇ bis C₂₀-Arylalkyl, C₇ bis C₂₀-Alkylaryl bedeuten oder R⁵ und R⁶ einen gesättigten Ring mit 5 bis 6 Kohlenstoff- oder Heteroatomen bilden, insbesondere mit den folgenden Aldehyden und Ketonen: Formaldehyd, Methylethylketon, Methylpropylketon, Diethylketon, Cyclohexanon, Acetophenon, in Gegenwart von der Reaktionsmischung hinzugefügtem Wasser. Bevorzugt wird Aceton verwendet.

Schon die Zugabe geringer Wassermengen bewirkt eine Erhöhung der Selektivität. Als günstig stellt sich ein Wassergehalt im Bereich der 0,1- bis 2,0-fachen, insbesondere bis 1,5-fachen molaren Menge des eingesetzten Ketons dar.
Das Phenol(derivat)-Keton/Aldehyd-Verhältnis bewegt sich gleichzeitig in einem Bereich von 3 : 1 bis 25 : 1. Wesentlich ist dabei ein Wassergehalt von 0,1 bis 30 Gew.-% Wasser, insbesondere 1 bis 10 Gew.-%, bezogen auf die Reaktionsmischung aus Phenolderivaten und Carbonylverbindungen.

Die Kondensation von Phenolderivaten gemäß Formel (IX) mit Carbonylverbindungen gemäß Formel (X) kann kontinuierlich (Festbettreaktor) oder diskontinuierlich (Slurryreaktor) unter den folgenden Bedingungen durchgeführt werden.

Im diskontinuierlichen Betrieb verwendet man im allgemeinen pro Moläquivalent eingesetzter Carbonylverbindung 50 bis 250 g, bevorzugt 100 bis 150 g Trockensubstanz des erfindungsgemäß verwendeten Katalysators. Das molare Verhältnis der Ausgangsverbindungen Phenol (I) und Aceton (II) beläuft sich auf 3 : 1 bis 25 : 1, bevorzugt 5 : 1 bis 15 : 1. Die Reaktionstemperaturen liegen im Bereich von 40 bis 150°C, vorzugsweise oberhalb des Schmelz- bzw. Erstarrungspunktes der beteiligten Komponenten, bevorzugt zwischen 50 und 70°C. Die Reaktionszeiten bewegen sich im allgemeinen zwischen 10 Minuten und 24 Stunden, bevorzugt zwischen 30 Minuten und 6 Stunden.

Erfindungsgemäß sind Reaktionstemperaturen von 50 bis 70°C, insbesondere 55 bis 60°C, besonders bevorzugt. Man erzielt auf diesem Wege sehr hohe Selektivitäten in bezug auf die p/p-Produktausbeute.

Ohne Zusatz von Wasser müßte man mit der Auskristallisation des Reaktionsgemisches und damit der Verstopfung des Reaktors rechnen.

Im kontinuierlichen Verfahren wird im allgemeinen ein Gemisch aus Phenolen gemäß Formel (VII) und Carbonylverbindungen gemäß Formel (VIII) im Verhältnis von 3 : 1 bis 25 : 1, bevorzugt 5 : 1 bis 15 : 1 und Wasser durch einen Festbettreaktor geleitet, der mit bis zu 90 % seines Volumens mit dem erfindungsgemäß einzusetzenden Katalysator gefüllt ist. Die Temperatur im Reaktor liegt zwischen 40 und 150°C, bevorzugt zwischen 50 und 70°C. Die WHSV (weight hourly space velocity) bewegt sich im Bereich von 0,25 bis 24 h⁻¹, bevorzugt werden zwischen 0,5 und 2 h⁻¹. Das Reaktionsgemisch kann dabei den Reaktor von oben nach unten als auch von unten nach oben durchströmen.

Anschließend wird das Produktgemisch der Aufarbeitung und Reinigung zugeführt, die - ebenso wie bei einem Produktstrom aus dem diskontinuierlich betriebenen Slurryreaktor - durch übliche Methoden wie Destillation und Kristallisation erfolgt.

Das erfindungsgemäß erhaltene Produkt mit einem hohen p/p-Anteil vereinfacht die Aufarbeitung, da Umkristallisationsschritte zur Abtrennung von Nebenprodukten wegen der verbesserten Selektivität eingespart werden können. Dies gilt insbesondere, wenn man z. B. erfindungsgemäß eine Selektivität von mehr als 45, insbesondere ≥ 50 bis 60 und mehr erreicht, ausgedrückt als Molverhältnis von z. B.p,p BPA zu o,p BPA (Bisphenol-A). Diese Isomerenverhältnisse erzielt man erfindungsgemäß im allgemeinen bei unveränderter Produktivität.

Bekannte Verfahren arbeiten mit einem hohen Überschuß an Phenol, um sowohl eine ausreichend hohe Selektivität, d. h. eine geringe Nebenproduktbildung und auch eine niedrige Farbzahl des Produktes zu gewährleisten, als auch ein Auskristallisieren des höher schmelzenden Bisphenols im Reaktor zu verhindern. Das bei der Kondensationsreaktion von Ketonen mit Phenolen entstehende Reaktionswasser wird in aufwendigen mehrstufigen Reinigungsschritten abgetrennt, da die Anwesenheit von Wasser im recyclierten Phenol zu einer deutlichen Aktivitätseinbuße des Katalysators bei bestenfalls nahezu unveränderter, in der Regel jedoch schlechterer Selektivität führt.

Ziel der vorliegenden Erfindung ist es, ein Verfahren zu finden, mit dem Bisphenole hoher Reinheit und insbesondere hohen Isomerenverhältnissen von p,p-/o,p-Produkt hergestellt werden können. Das erfindungsgemäße Verfahren ermöglicht die Herstellung von Bisphenolen mit Isomerenverhältnissen von p,p-/o,p-Produkt von 50 bis hoch zu 60 bei nahezu unveränderter Produktivität und höher bei reduzierter Produktivität.

Überraschenderweise führt die Zugabe von Wasser zu dem Reaktionsgemisch an sich schon zu einer Erhöhung der Selektivität der Reaktion bei nahezu unveränderter Aktivität des Katalysators. Zusätzlich führt die Erhöhung des Wasseranteils im Rohproduktstrom zu einer höheren Löslichkeit und einer reduzierten Neigung zur Auskristallisation des gebildeten Bisphenols. Dieses ermöglicht eine Durchführung der Reaktion bei niedrigeren Temperaturen, bei denen nochmals eine Steigerung der Selektivität beobachtet wird. Ein weiterer Vorteil des erfindungsgemäßen Verfahrens besteht darin, daß auf die aufwendige Trocknung des recyclierten Phenols verzichtet und das wasserenthaltende, destillativ abgetrennte Phenol direkt wieder eingesetzt werden kann.

Als Katalysatoren setzt man Katalysatoren in Form sulfonat- und mercaptogruppenhaltigen Organopolysiloxanen ein, bestehend aus Einheiten der Formel

[O_{3/2}-Si-R¹-SO₃-]ₓM^{x+} (I)

wobei R¹ eine lineare oder verzweigte Alkylengruppe mit 1 bis 12 C-Atomen, eine Cycloalkylengruppe mit 5 bis 8 C-Atomen oder eine Einheit der allgemeinen Formel 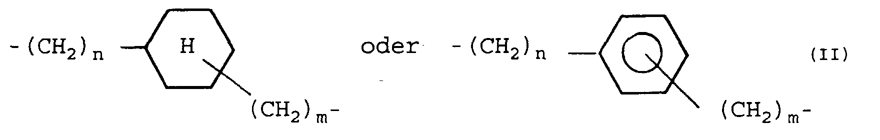 darstellt, in der n bzw. m eine Zahl von 0 bis 6 ist und die Zahl der silicium- bzw. schwefelständigen Methylengruppen angibt,
M gleich H⁺ oder gegebenenfalls auch NH₄⁺ oder ein Metallion mit einer Wertigkeit von x gleich 1 bis 4 ist, und Einheiten der Formel

O_{3/2}-Si-R²-SH (III)

mit R² ≙ R¹, wobei die freien Valenzen der an das Siliciumatom gebundenen Sauerstoffatome durch die Siliciumatome weiterer Gruppen der Formel (I) und/oder durch die Metallatomsauerstoffgruppen der vernetzenden Brückenglieder der Formeln

SiO_{4/2} (IV)

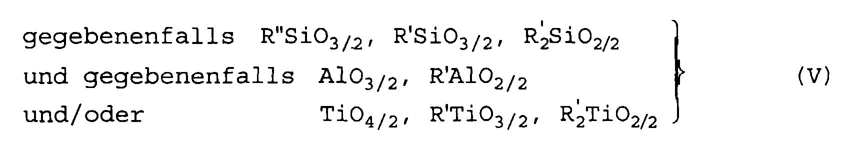

abgesättigt sind,
wobei R' eine Methyl- oder Ethylgruppe ist,
R'' Phenyl bedeutet oder eine lineare oder verzweigte C₂-C₁₂-Alkylgruppe und das Verhältnis der Gruppen der Formel (I) zu Gruppen der Formel (III) von 10 : 1 bis 1 : 10, das Verhältnis der Gruppen der Formel (I) zu Gruppen der Formel SiO_{4/2} von 1 : 3 bis 1 : 20, und
das Verhältnis der Gruppen der Formel SiO_{4/2} zu Gruppen der Formel (V) von 1 : 0 bis 1 : 0,5 reicht,
durchführt und das gewünschte Produkt abtrennt.

R¹ und R² besitzen dabei in den Formeln (I) und (II) bevorzugt dieselbe Bedeutung,
wobei R¹ und R² insbesondere den Propylenrest und M^{x+} H⁺ darstellt.

Je nach Vorbehandlung besitzen die kugelig geformten erfindungsgemäß eingesetzten Polysiloxane einen Teilchendurchmesser von 0,01 bis 3,0, vorzugsweise 0,05 bis 2,0 mm, eine spezifische Oberfläche von > 0 bis 1000, vorzugsweise > 0 bis 700 m²/g und eine Schüttdichte von 50 bis 1000 g/l, vorzugsweise 100 bis 800 g/l. Die einstellbaren Porendurchmesser liegen im Bereich von > 0 bis über 1000 nm.
Bevorzugt eingesetzt werden Katalysatoren der Typen:

9 SiO₂ · SiO_{3/2}(CH₂)₃SO₃H · 4 SiO_{3/2}(CH₂)₃SH

9 SiO₂ · SiO_{3/2}(CH₂)₃SO₃H · 2 SiO_{3/2}(CH₂)₃SH

10 SiO₂ · SiO_{3/2}(CH₂)₃SO₃H · 3 SiO_{3/2}(CH₂)₃SH

10 SiO₂ · 2 AlO_{3/2} · SiO_{3/2}CH₂-C₆H₄-CH₂SO₃H · 2 SiO_{3/2}(CH₂)₃SH

36 SiO₂ · 4 SiO_{3/2}(CH₂)₃SO₃H · SiO_{3/2}(CH₂)₃SH

9 SiO₂ · SiO_{3/2}(CH₂)₃SO₃H · 3 SiO_{3/2}(CH₂)₃SH · SiO_{3/2}(CH₂)₃CH₃

6 SiO₂ · SiO_{3/2}(CH₂)₃SO₃H · 2 SiO_{3/2}(CH₂)₃SH

Als Katalysatoren geeignet sind ebenso sulfonat- und mercaptogruppenhaltige anorganische Trägermaterialien wie sie z. B. entstehen, wenn man oxidische und silikatische Feststoffe mit Organosiliciumverbindungen der allgemeinen Formel

[(RO)_{y}Si-R¹-SO₃⁻]ₓM^{x+} (XI)

und der allgemeinen Formel

(RO)_{y}Si-R²-SH (XII)

modifiziert.

### Beispielteil:

1. Einfluß des Wassers auf die Selektivität und Aktivität bei einer konstanten Temperatur
Ein gemäß DE 195 36 363.9 hergestellter Katalysator der Zusammensetzung

O_{3/2}Si-CH₂ CH₂ CH₂S0₃H · 4 O_{3/2}Si-CH₂ CH₂ CH₂SH · 9 SiO₂

wird in einem beheizten Rohrreaktor kontinuierlich von unten mit einer Phenol/Aceton/Wasser Schmelze durchströmt.
Dabei wurden folgende Versuchsparameter eingestellt:
- Temperatur:: 60 °C
- Molverhältnis Phenol/Aceton:: 10:1
- Masse Katalysator:: 30 g
- WHSV:: 0,7 h⁻¹

Die folgende Tabelle gibt die erzielten Selektivitäten (Isomerenverhältnis) sowie den Acetonumsatz an:

| Wassergehalt [mol H₂O/ mol Aceton] | Selektivität [p,p BPA/o,p BPA ] | WHSV [h⁻¹] | Umsatz Aceton [%] |
|---|---|---|---|
| 0/1 | 41,2 | 0,7 | 99,6 |
| 1/1 | 60,2 | 0,7 | 87,3 |

Durch den Zusatz von Wasser zu der Phenol/Aceton-Schmelze wird die Selektivität drastisch gesteigert. Der Acetonumsatz geht nur geringfügig zurück .
2. Einfluß der Temperatur auf die Selektivität bei gleichbleibendem Wassergehalt
Ein gemäß DE 195 36 363.9 hergestellter Katalysator der Zusammensetzung

O_{3/2}Si-CH₂ CH₂ CH₂S0₃H · 2 O_{3/2}Si-CH₂ CH₂ CH₂SH · 9 SiO₂

wird wie in Beispiel 1 in einem beheizten Rohrreaktor kontinuierlich von unten mit einer Phenol/Aceton/Wasser Schmelze durchströmt. Folgende Versuchsparameter wurden eingestellt:
- Molverhältnis Phenol/Aceton:: 10:1
- Molverhältnis H₂O/Aceton:: 1:1
- Masse Katalysator:: 38 g
- WHSV:: 0,68 h⁻¹

Die folgende Tabelle zeigt den Einfluß der Temperatur auf die Selektivität. Hierbei ist insbesondere zu bemerken, daß ohne Wasserzusatz der Reaktor bei den beiden niedrigeren Temperaturen nicht betrieben werden könnte, da dann unmittelbar Auskristallisation des Produktes und Verstopfung des Rohrreaktors auftritt.

| Temperatur [°C] | Selektivität [ p,p BPA/ o,p BPA ] | Umsatz Aceton [%] |
|---|---|---|
| 55 | 60,3 | 42,3 |
| 60 | 55,6 | 53,5 |
| 65 | 43,6 | 59,1 |

3. Einfluß der Stöchiometrie des eingesetzten Katalysators (Sulfonsäure/Mercaptogruppenverhältnis)
Bei Anwesenheit von Wasser in der Phenol/Aceton-Schmelze ist eine deutliche Abstufung des erzielten Isomerenverhältnis von der Katalysatorzusammensetzung zu beobachten. Dies ist bei Abwesenheit von Wasser weitaus geringer ausgeprägt. Folgende Versuchsparameter wurden eingestellt:
- Temperatur:: 60 °C
- Molverhältnis Phenol/Aceton:: 10:1
- Molverhältnis H₂O/Aceton:: 1:1
- WHSV:: 0,7 - 0,8 h⁻¹

| Katalysatorzusammensetzung | Selektivität [p,p BPA/o,p BPA ] |
|---|---|
| O_{3/2}Si-CH₂ CH₂ CH₂S0₃H · 4 O_{3/2}Si-CH₂ CH₂ CH₂SH · 9 SiO₂ | 60,2 |
| O_{3/2}Si-CH₂ CH₂ CH₂S0₃H · 2 O_{3/2}Si-CH₂ CH₂ CH₂SH · 9 SiO₂ | 55,6 |

4. Vergleichsbeispiel 1 unter Verwendung eines Polystyrol-Ionenaustauschers
Ein Kationenaustauscher (Amberlyst 15) wurde gemäß der in Beispiel 2 der Offenlegungsschrift DE 36 19 450 vom 17.12.1987 beschriebenen Methode mit Mercaptoethylamin modifiziert und in gleicher Weise wie in den obigen Beispielen beschrieben getestet.
Folgende Versuchsbedingungen wurden eingestellt:
- Molverhältnis Phenol/Aceton:: 10:1
- Masse Katalysator:: 35 g
- WHSV:: 0,68 h⁻¹
- Temperatur:: 60 °C

| Wassergehalt [mol H₂O/ mol Aceton] | Selektivität [p,p BPA/o,p BPA ] | Umsatz Aceton [%] |
|---|---|---|
| 1/1 | 31,8 | 17,2 |
| 0/1 | 42,1 | 59,0 |

Im Gegensatz zu den im erfindungsgemäßen Verfahren eingesetzten Katalysatoren zeigt sich bei Zusatz von Wasser ein deutlicher Aktivitätsverlust wie auch eine Verschlechterung der Selektivität.
5. Vergleichsbeispiel 2 unter Verwendung eines Polystyrol-Ionenaustauschers
Ein Polystyrol-Kationentauscher (Dowex® 50 W X 8) wurde gemäß Beispiel 1 des US Patents 3,634,341 vom 11.01.1972 mit 2,2 Dimethylthiazolidin modifiziert und mit der hier in diesem Patent beschriebenen Apparatur mit und ohne Wasserzusatz getestet.
Folgende Versuchsbedingungen wurden eingestellt:
- Molverhältnis Phenol/Aceton:: 10:1
- Masse Katalysator:: 54 g
- WHSV:: 0,55 h⁻¹
- Temperatur:: 65 °C

| Wassergehalt [mol H₂O/ mol Aceton] | Selektivität [ p,p BPA/ o,p BPA ] | Umsatz Aceton [%] |
|---|---|---|
| 1/1 | 21,8 | 38,8 |
| 0/1 | 23,9 | 91,2 |

Auch in diesem Fall nimmt mit der Zugabe von Wasser zu der Phenol/Aceton-Schmelze - wie in Beispiel 4 die Selektivität und auch die Aktivität des Katalysators deutlich ab.

## Patentansprüche

1. Verfahren zur selektiven Herstellung von 2,2'-Bis(4-hydroxyphenyl)propanen der allgemeinen Formel 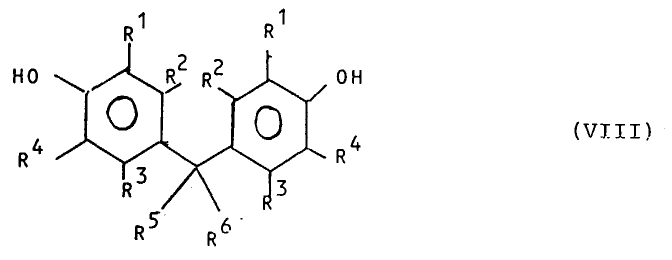 aus Phenolen der allgemeinen Formel 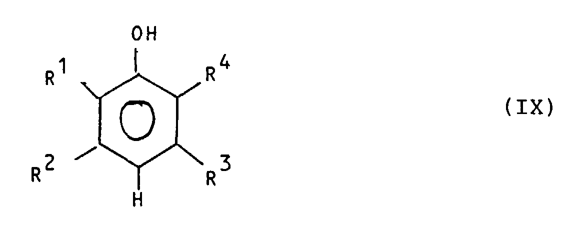 in der R¹, R², R³ und R⁴ unabhängig voneinander für Wasserstoff, C₁- bis C₄-Alkylgruppen, aromatische Substituenten oder Halogene wie F, Cl, Br und I stehen, durch Umsetzung mit Carbonylverbindungen der allgemeinen Formel 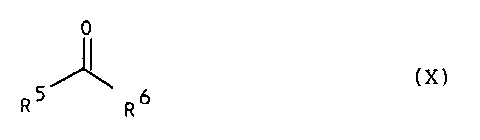 in der R⁵ und R⁶ unabhängig voneinander Wasserstoff, C₁ bis C₆-Alkyl, C₆ bis C₁₀-Cycloalkyl, C₃ bis C₁₄-Aryl, C₇ bis C₂₀-Arylalkyl, C₇ bis C₂₀-Alkylaryl bedeuten oder R⁵ und R⁶ einen gesättigten Ring mit 5 bis 6 Kohlenstoff- oder Heteroatomen bilden, in Gegenwart eines sulfonat- und mercaptogruppenhaltigen Organopolysiloxans oder mit diesen Gruppen modifizierten anorganischen Trägermaterialien,
**dadurch gekennzeichnet,**
daß man der Reaktionsmischung Wasser zusetzt.

2. Verfahren gemäß Anspruch 1,
**dadurch gekennzeichnet,**
daß man der Reaktionsmischung Wasser und Carbonylverbindung gemäß Formel (X) im molaren Verhältnis von 0,1 : 1 bis 2,0 : 1 einsetzt.

3. Verfahren gemäß den Ansprüchen 1 und 2,
**dadurch gekennzeichnet,**
daß man ein Gemisch aus Phenolen gemäß Formel (IX) und Carbonylverbindungen gemäß Formel (X) im molaren Verhältnis von 3 : 1 bis 25 : 1 und Wasser kontinuierlich durch einen Festbettreaktor leitet.

4. Verfahren gemäß den Ansprüchen 1 bis 3,
**dadurch gekennzeichnet,**
daß man das Gemisch mit einer WHSV von 0,25 bis 24 h⁻¹ durch den Festbettreaktor leitet.

5. Verfahren gemäß den Ansprüchen 1 und 2,
**dadurch gekennzeichnet,**
daß man das Verfahren diskontinuierlich durchführt, wobei man ein Gemisch aus Phenolen gemäß Formel (IX) und Carbonylverbindungen gemäß Formel (X) im molaren Verhältnis von 3 : 1 bis 25 : 1 und Wasser in Gegenwart von 50 g bis 250 g Trockensubstanz des Katalysators gemäß Anspruch 1 miteinander umsetzt, bezogen auf ein Moläquivalent der Carbonylverbindung.

6. Verfahren gemäß den Ansprüchen 1 bis 5,
**dadurch gekennzeichnet,**
daß das Verhältnis von SH-Gruppen zu SO₃H-Gruppen auf dem Katalysator zwischen 1 : 10 und 10 : 1 beträgt.

7. Verfahren gemäß den Ansprüchen 1 bis 6,
**dadurch gekennzeichnet,**
daß das Verhältnis von SH-Gruppen zu SO₃H-Gruppen auf dem Katalysator zwischen 2 : 1 bis 4 : 1 liegt.

8. Verfahren gemäß einem oder mehreren der vorgehenden Ansprüche,
**dadurch gekennzeichnet,**
daß man die Reaktion bei einer Temperatur von 50 bis 70°C durchführt.

9. Verfahren gemäß Anspruch 8,
daß man die Reaktion bei einer Temperatur von 55 bis 60°C durchführt.
